(19) Europäisches Patentamt
European Patent Office
Office européen des brevets

(11) Publication number: **0 440 165 A2**

(12) **EUROPEAN PATENT APPLICATION**

(21) Application number: **91101142.7**

(22) Date of filing: **29.01.91**

(51) Int. Cl.5: **C08G 63/06**, C12P 7/62, //(C12P7/62,C12R1:05)

(30) Priority: **29.01.90 JP 18502/90**

(43) Date of publication of application: **07.08.91 Bulletin 91/32**

(84) Designated Contracting States: **AT BE DE DK FR GB IT NL**

(71) Applicant: **SHOWA DENKO KABUSHIKI KAISHA**
**13-9, Shiba Daimon 1-chome**
**Minato-ku, Tokyo 105(JP)**

(72) Inventor: **Nishimura, Atsuo, Showa Denko K.K. Cent. Res. Lab.**
**Engineering Res. Cent., 5-1, Ohkawa-cho**
**Kawasaki-ku,Kawasaki-shi, Kanagawa(JP)**
Inventor: **Imanaka, Mamoru, Showa Denko K.K. Cent. Res. Lab.**
**Engineering Res. Cent., 5-1, Ohkawa-cho**
**Kawasaki-ku,Kawasaki-shi, Kanagawa(JP)**
Inventor: **Niitsu, Hiromi, c/o Showa Denko K.K.**
**Oita Research Lab., 2, Oaza Nakanosu**
**Oita-shi, Oita(JP)**
Inventor: **Nishioka, Masaaki, c/o Showa Denko K.K.**
**Seikagaku Kenkyusho, 2-24-25, Tamagawa**
**Ota-ku, Tokyo(JP)**
Inventor: **Gotoh, Katsuhiko, c/o Showa Denko K.K.**
**13-9, Shiba Daimon 1-chome**
**Minato-ku, Tokyo(JP)**

(74) Representative: **Strehl, Schübel-Hopf, Groening**
**Maximilianstrasse 54 Postfach 22 14 55**
**W-8000 München 22(DE)**

(54) **Biodegradable or biocompatible copolymer and process for producing same.**

(57) A random copolymer comprising, as repeating units,

(i) 50 to 97 mol% of 3-hydroxybutyrate unit (3HB) having the formula (I):

$$-O-\underset{\substack{|\\CH_3}}{CH}CH_2CO- \qquad (I)$$

(ii) 1 to 25 mol% of 3-hydroxyvalerate unit (3HV) having the formula (II):

$$-O-\underset{\substack{|\\CH_2CH_3}}{CH}CH_2CO- \qquad (II)$$

(iii) 1 to 15 mol% of 3-hydroxypropionate unit (3HP) having the formula (III):

$-OCH_2CH_2CO-$ (III)

and

(iv) 1 to 10 mol% of 5-hydroxyvalerate unit (5HV)

EP 0 440 165 A2

$- OCH_2\ CH_2\ CH_2\ CH_2\ CO -$ (IV)

wherein the total of the units 3HB, 3HV, 3HP and 5HV is 100 mol%, and having a weight average molecular weight within the range of from 10,000 to 2,500,000.

# BIODEGRADABLE OR BIOCOMPATIBLE COPOLYMER AND PROCESS FOR PRODUCING SAME

## BACKGROUND OF THE INVENTION

### 1. Field of the Invention

The present invention relates to a novel copolymer produced by using a microorganism, and a process for producing the same. More specifically, it relates to a novel four-unit copolymer having an excellent moldability and comprising 3-hydroxybutyrate units (hereinafter sometimes abbreviated as 3HB), 3-hydroxyvalerate units (hereinafter sometimes abbreviated as 3HV), 3-hydroxypropionate units (hereinafter sometimes abbreviated as 3HP), and 5-hydroxyvalerate units (hereinafter sometimes abbreviated as 5HV), and a process for producing the same.

### 2. Description of the Related Art

Since poly-3-hydroxybutyrate (PHB) is accumulated within cells of a large number of microorganisms, as an energy storing substance, and is a thermoplastic polymer exhibiting an excellent biodegradability and biocompatibility, it has attracted attention as a "clean" plastic which protects the environment. Particularly, under the present situation in which synthetic plastics are a serious social problem, from the standpoints of environmental pollution and resource circulation, PHB also has attracted attention as a biopolymer which does not depend on petroleum.

For example, PHB can be applied for medical materials such as surgical thread or broken bone setting materials, hygienic articles such as diapers or sanitary articles, agricultural or horticultural materials such as multi films, slow release chemicals, fishery materials such as fishing nets, packaging materials, and in many other fields.

Nevertheless, PHB has mechanical problems in that it has a high crystallinity (70% or more) and a poor flexibility and can be worked only with difficulty, since it is thermally decomposed at a temperature of the melting point (180 °C) thereof or higher, and has a poor impact resistance. Further, beoause of high production costs thereof, PHB has not been industrially produced on a practical scale.

Recently, studies have been made into a copolymer basically comprising a 3-hydroxybutyrate (3HB) unit and a process for the production thereof using Alcaligenes eutrophus.

For example, a copolymer comprising 3HB and 3-hydroxyvalerate units (3HV) is disclosed in (e.g., Japanese Unexamined Patent Publications (Kokai) Nos. 57-150393, 58-69224, 59-220192, 63-269989 and 64-69622.

The introduction of the 3HV component into the copolymer lowers the crystallinity and improves the flexibility of the same. Nevertheless, this copolymer still has problems in that the thermal resistance is poor for a resin for practical use, because the melting temperature is 85 to 106 °C; valeric acid, which is expensive, is used as a carbon source (Japanese Unexamined Patent Publication (Kokai) Nos. 63-269989 and 64-69622); and the production process is complicated (Japanese Unexamined Patent Publication (Kokai) No. 59-220192).

The terpolymer (or three unit copolymer) wherein, in addition to the above-mentioned 3HB and 3HV, a 5-hydroxyvalerate unit (5HV) is introduced as a third component is disclosed in Japanese Unexamined Patent Publication (Kokai) No. 64-48820, and the terpolymer wherein a 3-hydroxypropionate unit (3HP) is introduced as a third component is disclosed in Japanese Unexamined Patent Publication (Kokai) No. 58-69224.

The former terpolymer has a problem of the thermal resistance thereof, because it has a low melting temperature of 101 °C. Furthermore, the 5-chloro-valeric acid used as a carbon source is expensive, the yield of the microorganism cells is low (4.2 to 4.7g/ℓ), and the polymer content in the microorganism cells is low. Therefore, this terpolymer cannot be practically used.

The latter terpolymer has a high melting temperature of 170 to 172 °C, and at a temperature higher than this temperature, it is easily thermally decomposed, and therefore, problems arise during melt molding thereof. Further, the microorganism cell yield is low (4.5 to 7.6 g/ℓ).

Japanese Unexamined Patent Publication (Kokai) Nos. 64-48821 and (Heisei) 1-222788 disclose a copolymer to which, as a copolymer component, a 4-hydroxybutyrate unit (4HB) is further introduced. This copolymer has drawbacks such that the melting temperature thereof is 156 to 159 °C, thermal decomposition during thermal melt molding thereof occurs, and the microorganism cell yield is low, i.e., 2.7 to 8.5 g/ℓ. Therefore, this copolymer is not practically used.

As described above, a practical thermoplastic copolymer having a required flexibility, which is not thermally decomposed during molding and is sufficiently resistant to the heat of, for example, thermal disinfection, has an excellent moldability, and is produced by microorganisms having a biodegradability and biocompatibility characteristics, as well as a process for producing the same, are not conventionally known.

## SUMMARY OF THE INVENTION

Accordingly, the objects of the present invention are to eliminate the above-mentioned disadvantages of the prior art and to provide a practical copolymer produced by a microorganism and having biodegradability or biocompatibility characteristics, and further, has the required flexibility, is not thermally decomposed during molding, is sufficiently resistant to the heat of, for example, thermal disinfection, and has excellent moldability.

Other objects and advantages of the present invention will be apparent from the following description.

In accordance with the present invention, there is provided 1) a random copolymer comprising, as repeating units,

(i) 50 to 97 mol% of a 3-hydroxybutyrate unit (3HB) having the formula (I):

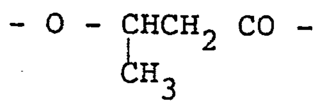

(I)

(ii) 1 to 25 mol% of a 3-hydroxyvalerate unit (3HV) having the formula (II):

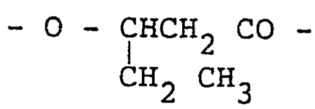

(II)

(iii) 1 to 15 mol% of a 3-hydroxypropionate unit (3HP) having the formula (III):

$-OCH_2CH_2CO-$ (III)

and

(iv) 1 to 10 mol% of a 5-hydroxyvalerate unit (5HV) having the formula (IV):

$-OCH_2CH_2CH_2CH_2CO-$ (IV)

wherein the total of the units 3HB, 3HV, 3HP and 5HV is 100 mol%, and having a weight average molecular weight of from 10,000 to 2,500,000.

In accordance with the present invention, there is also provided a process for producing the above-mentioned random copolymer, comprising culturing a microorganism capable of producing a polyhydroxyalkanoate under at least one restricted condition of the amount of nitrogenous and/or phosphorous source, in the presence of δ-valerolactone, 1,5-pentanediol and/or mono- or bis-carboxylic acid ester of 1,5-pentanediol, to form and accumulate a random copolymer comprising 3-hydroxybutyrate units (3HB), 3-hydroxyvalerate units (3HV), 3-hydroxypropionate units (3HP) and 5-hydroxyvalerate units (5HV) within the microorganism cells, followed by recovering the same.

## DESCRIPTION OF THE PREFERRED EMBODIMENTS

The present inventors have conducted extensive studies and research into the obtaining of a copolymer having various properties, particularly an excellent moldability, produced by microorganisms having biodegradability and biocompatibility characteristics. As a result it was found that, by using δ-valerolactone, 1,5-pentanediol or the carboxylic acid ester thereof, a microorganism is cultured which is conventionally known to have a polyhydroxyalkanoate production ability, whereby a four-unit random copolymer comprising 3HB, 3HV, 3HP and 5HV units is generated and accumulated within cells of the microorganism, and that the four-unit random copolymer thus obtained has an excellent moldability. Accordingly, the above-mentioned problems can be overcome by the present invention.

The present invention will be illustrated in detail as follows.

Microorganism

Any microorganisms capable of producing polyhydroxyalkanoates such as polyhydroxybutyrate (PHB) can be used. Practically, for example, Alcaligenes faecalis, Alcaligenes ruhlandii, Alcaligenes latus, Alcaligenes aquamarinus and Alcaligenes eutrophus can be used.

Note, a mutant strain obtained by an artificial mutation treatment of these strains and a strain similar to said strains but obtained by a gene -engineering technique also can be used.

As typical examples of the strain belonging to these genera, mention may be made of Alcaligenes faecalis ATCC 8750, Alcaligenes ruhlandii ATCC 15749, Alcaligenes latus ATCC 29712, Alcaligenes aquamarinus ATCC 14400 and Alcaligenes eutrophus H-16 ATCC 17699, Alcaligenes eutrophus NCIB 11597, NCIB 11598, NCIB 11599 and NCIB 11600, which are the mutant strains of the H-16 strain.

Among the above, in view of the practical use thereof, Alcaligenes eutrophus H-16 ATCC 17699 and Alcaligenes eutrophus NCIB 11599 are particularly desirable.

The bacteriological properties of these microorganisms belonging to the genus Alcaligenes are described in, for example, "BERGEY's MANUAL OF DETERMINATIVE BACTERIOLOGY, Eighth Edition, The Williams & Wilkins Company/Baltimore", and further, the bacteriological properties of Alcaligenes eutrophus H-16 are described in, for example, "J. Gen. Microbiol., 115, 185-192 (1972)", respectively.

Cultivation Method

As in the conventional methods, these microorganisms are cultured according to the two step process. These two steps are a first cultivation step for the growth of microorganism cells, and a second cultivation step for the generation and accumulation of a copolymer within microorganism cells while restricting nitrogen and phosphorus.

First step cultivation

For the cultivation in the first step, the conventional cultivation method of growing microorganisms is applicable, i.e., a medium and cultural conditions in which the microorganism to be used can be grown may be employed.

The culture medium components are not particularly limited, provided that they are substances which can be utilized by the microorganism to be used, but in practice, as the carbon sources, there may be employed synthetic carbon sources such as methanol, ethanol and acetic acid, inorganic carbon sources such as carbon dioxide, natural products such as yeast extract, molasses, peptone and meat extract, saccharides such as arabinose, glucose, mannose, fructose and galactose, and sorbitol, mannitol and inositol. As the nitrogen sources, for example, inorganic nitrogen compounds such as ammonia, ammonium salts, nitrates, and/or organic nitrogen containing compounds such as urea, corn steep liquor, casein, peptone, yeast extract, meat extract may be employed.

The inorganic components can be selected, for example, from calcium salts, magnesium salts, potassium salts, sodium salts, phosphoric acid salts, manganese salts, zinc salts, iron salts, copper salts, molybdenum salts, cobalt salts, nickel salts, chromium salts, boron compounds, and iodine compounds.

If necessary, vitamins can be employed.

The cultural conditions are not particularly limited, but the temperature is, for example, about 20 to 40°C, preferably about 25 to 35°C, and the pH is, for example, about 6 to 10, preferably about 6.5 to 9.5. The aerobic cultivation is carried out under these conditions.

When the cultivation is carried out in a range outside such conditions, the growth of the microorganism may be relatively poor, but the cultivation in a range outside such conditions will not be obstructed.

The cultivation system may be either batchwise or continuous.

Second Cultivation Step

The cells obtained in the cultivation by the first step are further cultured under conditions whereby the amount of nitrogenous and/or phosphorous source is restricted.

More specifically, the microorganism cells are recovered by separation, by a conventional solid-liquid

separation means such as filtration and centrifugation, from the culture broth obtained in the first step, and the cells thus obtained are subjected to cultivation in the second step. Alternatively, in the cultivation in the previous step, nitrogenous and/or phosphorous source is substantially depleted, and the culture broth can be migrated to cultivation in the second step without a recovery by separation of the cells to be cultured therein.

The cultivation in the second step is the same as the cultivation in the first step, except that substantially no nitrogenous and/or phosphorous source is contained in the culture medium or the culture broth, and δ-valerolactone, 1,5-pentanediol and/or mono- or bis-carboxylic acid ester of 1,5-pentanediol are contained as the specified carbon source.

This carbon source is contained in the culture medium as the culture broth of the second cultivation stage. In the latter case, the carbon source may be added at any time during the cultivation, from the initial stage through to the end stage of the cultivation, but an addition at the initial stage is preferable.

The carbon source may be used in an amount which can generate a copolymer and does not inhibit the growth of a microorganism, and usually is used in an amount of about 5 to 200 g, preferably 10 to 160 g, per 1 ℓ of a culture medium or a culture broth.

In the cultivation in this second step, δ-valerolactone, 1,5-pentanediol and/or mono- or bis-oarboxylic acid esters of 1,5-pentanediol is used alone or in any mixture thereof.

As the mono- or bis-carboxylic acid ester of 1,5-pentanediol, lower carboxylic acid suoh as formate, acetate, propionate and butyrate may be used.

Other carbon sources utilizable by the microorganism employed, such as glucose, fructose, methanol, ethanol, acetic acid, propionic acid, n-butyric acid, and lactose, also can be permitted to coexist in small amounts. For example, when propionic acid is permitted to coexist therein, the ratio of 3HV component can be easily controlled. In this case, although the amount of, for example, propionic acid is not particularly limited, preferably it is used in an amount of about 0.5 to 70% by weight, on the basis of the main carbon source specified above.

<u>Separation of Microorganism Cells and Copolymer</u>

From the culture broth thus obtained, the microorganism cells are recovered by separation, by a conventional solid-liquid separation means such as filtration and centrifugation, and the cells are washed and dried to obtain dry cells. The yield of the microorganism cells is about 30 to 100 g/ℓ, which is surprisingly large compared with that of the conventional processes of bi- or terpolymer (several grams/ℓ - ten to twenty grams/ℓ).

The copolymer formed is extracted from the dry cells or the wet cells in a conventional manner, for example, by extraction with an organic solvent such as chloroform, and to the extract is added, for example, a poor solvent which does not readily dissolve the copolymer such as hexane, to thereby precipitate the copolymer. Alternatively, separated and recovered microorganism cells are treated with chemicals such as sodium hypochlorite which can solubilize the microorganism cells, or with an enzyme and/or a surfactant, to thereby solubilize the cell membrane and the cytoplasm of the microorganism cells, and the copolymer granules are then recovered by separation by a conventional solid-liquid separation means such as filtration or centrifugal separation, followed by washing and drying the copolymer.

<u>Properties of Copolymer</u>

The copolymer thus obtained is a four-unit random copolymer wherein 3HB, 3HV, 3HP and 5HV components form ester bonds with each other.

The molecular weight and the ratio of each component can be controlled by changing the kind of microorganism used, and the kind or concentration of the carbon source (ℓ-valerolactone, 1,5-pentanediols and other carbon sources). Particularly, the copolymer comprises 50 to 97 mol%, preferably 65 to 97 mol%, of a 3HB component, 1 to 25 mol%, preferably 1 to 20 mol%, of a 3HV component, 1 to 15 mol%, preferably 1 to 10 mol%, of a 3HP component and 1 to 10 mol%, preferably 1 to 5 mol%, of a 5HV component (the total of each component is 100 mol%), has a weight average-molecular weight of 10,000 to 2,500,000, particularly 100,000 to 1,500,000, has a crystallinity of 50 to 65%, and is satisfactorily flexible. Further, the copolymer has a melting temperature of 120 to 130 °C, and therefore, can be fabricated at a temperature of 140 °C or less at which it is thermally decomposed, and in addition, has a heat-resistance high enough to allow a thermal disinfection to be effected.

<u>EXAMPLES</u>

The present invention will now be further illustrated by, but is by no means limited to, the following Examples.

Example 1

A copolymer was prepared by using Alcaligenes eutrophus ATCC 17699.

First cultivation step

First, 2 liters of the D medium having the following composition were introduced into a 5-liter jar fermenter, and the above-mentioned microorganism was aerobically cultured at a pH of 7 to 8 and a temperature of 30 to 35° C, for 20 hours. During the cultivation, the fructose was replenished in accordance with the consumption of the same.

The PH was automatically adjusted by using aqueous ammonia.

The medium D was composed of the following components:

| | |
|---|---|
| $(NH_4)_2SO_4$ | 4 g |
| $K_2HPO_4$ | 8 g |
| $KH_2PO_4$ | 1.2 g |
| NaCl | 0.5 g |
| $MgSO_4$ | 2.4 g |
| Mineral solution* | 20 ml |
| Fructose | 10 g |
| Deionized water | 1 ℓ |

The above-mentioned mineral solution* comprised the following components:

| | |
|---|---|
| $FeSO_4$ | 5 g |
| $CaCl_2$ | 10 g |
| $MnSO_4$ | 1 g |
| $CuSO_4$ | 100 mg |
| $ZnSO_4$ | 500 mg |
| $CoCl_2$ | 100 mg |
| $MoO_3$ | 1 mg |
| $H_3BO_3$ | 1 mg |
| Aqueous 0.1 N-HCℓ solution | 1 ℓ |

Second cultivation step

First, δ-valerolactone as a carbon source was gradually added, to an amount of 110 g/ℓ medium and the cultivation was effected at a pH of 7-8, at 30-35° C for 60 hours.

The pH was automatically adjusted by using an aqueous sodium hydroxide solution and an aqueous sulfuric acid solution.

Separation of Microorganism Cells

Microorganism cells were separated by centrifugal separation from the culture broth thus obtained, the resultant cells were vacuum dried, and 69 g/ℓ of dried cells was obtained.

Separation and recovery of copolymer

From the dry cells thus obtained, the copolymer was extracted with hot chloroform, precipitated by an addition of hexane to the extract, and the precipitates were subjected to filtration and drying, to give 33% by weight of the copolymer based on the weight of the dried microorganism cells.

Characteristics of copolymer

The composition, the molecular weight, the melting temperature, and crystallinity of the copolymer thus obtained were measured as described below:

| | |
|---|---|
| Composition: | $^{13}$C-NMR spectrum and $^{1}$H-NMR spectrum |
| Molecular weight: | Gel permeation chromatography (GPC) measurement |
| Melting temperature (Tm): | Differential scanning calorimeter (DSC) measurement |
| Crystallinity (Xc): | X-ray diffraction method |

The results are shown in Table 1.

The positions to which each carbon atom and hydrogen atom of the copolymer belongs were determined from $^{13}$C-NMR and $^{1}$H-NMR spectrum are shown in Table 2.

Table 3 shows the diad chain distribution of each component, estimated from the multiple-overlapped line resonance structure of a carbonyl carbon on the basis of Y. Doi. et al., Macromolecules, 19, 2860-2864-(1986). This chain distribution shows that the copolymer has a random copolymer chain distribution.

Examples 2 to 3

Example 1 was repeated except that, in the second cultivation step, 1,5-pentanediol 60 g/ℓ medium or 1,5-pentanediol bis (acetate) 80 g/ℓ medium was used as the carbon source.

The results are shown in Table 1.

Comparative Example 1

Example 1 was repeated except that, in the second cultivation step, acetic acid 19 g/ℓ medium was used as the carbon source.

Examples 4 to 6

Example 1 was repeated except that, in the second cultivation step, as the carbon source, propionic acid was made to coexist with δ-valerolactone, at the ratio shown in Table 4.

The results are shown in Table 4.

Table 1

| Example or Comparative Example | Carbon source | Weight of dry cells | Copolymer content in dry cells | Copolymer composition mol% | | | | Molecular weight*2 | | Melting temperature | Crystallinity |
|---|---|---|---|---|---|---|---|---|---|---|---|
| | Sort | g/ℓ*1 | % | [3HB] | [3HV] | [3HP] | [5HV] | Mw $\times 10^3$ | Mw/Mn | Tm °C | Xc % |
| Example 1 | δ-valerolactone | 69 | 33 | 92 | 2 | 5 | 1 | 420 | 1.4 | 126 | 62 |
| Example 2 | 1,5-pentanediol | 35 | 20 | 91 | 1 | 6 | 2 | 495 | 2.2 | 128 | 60 |
| Example 3 | 1,5-pentanediolbis (acetate) | 30 | 23 | 93 | 1 | 5 | 1 | 500 | 1.9 | 127 | 58 |
| Comparative Example 1 | Acetic acid | 11 | 67 | 100 | 0 | 0 | 0 | 600 | 1.7 | 178 | 70 |

*1 g/ℓ- Medium (culture broth)
*2 Mw: Weight average molecular weight
Mn: Number average molecular weight

Table 2

| Number *1 | ppm | |
|---|---|---|
| | C*2 | H*3 |
| 1 | 167.60 | —— |
| 2 | 40.67 | 2.46, 2.60 |
| 3 | 67.15 | 5.25 |
| 4 | 19.71 | 1.28 |
| 5 | 168.00 | —— |
| 6 | 9.30 | 2.46, 2.60 |
| 7 | 71.43 | 5.16 |
| 8 | 33.68 | 1.55, 1.66 |
| 9 | 21.35 | 0.90 |
| 10 | 168.70 | —— |
| 11 | 33.82 | 2.46, 2.60 |
| 12 | 59.58 | 4.33 |
| 13 | 171.60 | —— |
| 14 | 33.68 | 2.29 |
| 15 | 21.35 | 1.55, 1.66 |
| 16 | 27.95 | 1.55, 1.66 |
| 17 | 63.68 | 4.08 |

*1 Corresponds to the encircled numerals described in the following structural formula.

*2 Carbon atoms corresponding to each encircled numeral position described in the following structural formula, determined from $^{13}C$-NMR spectrum.

*3 Hydrogen atoms bound with carbon atoms corresponding to each encirculed numeral position described in the following structural formula determined from $^{1}H$-NMR spectrum.

Structural formula

$$-(3HB)_{X_1}-(3HV)_{X_2}-(3HP)_{X_3}-(5HV)_{X_4}-$$

3HB: 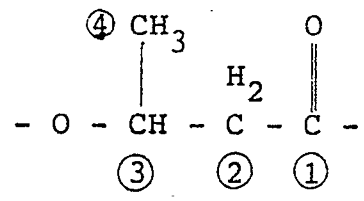

3HV: 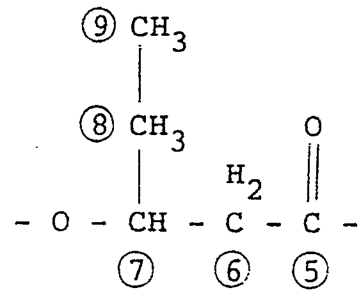

3HP: 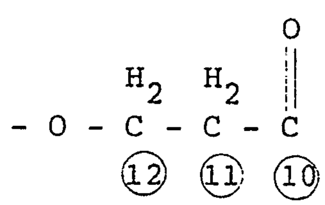

5HV: 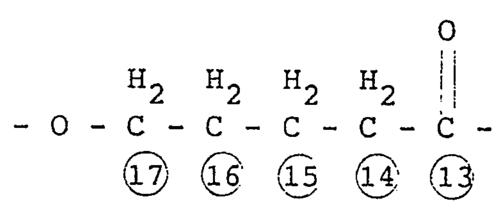

Table 3

| Di-add | | 3HB-3HB | 3HB-3HV | 3HV-3HV | 3HB-3HP | 3HP-3HB | 3HB-5HV | 3HP-3HP | 5HV-3HB |
|---|---|---|---|---|---|---|---|---|---|
| Chemical Shift (ppm) | | 167.6 | 167.8 | 168.0 | 168.3 | 168.1 | 170.8 | 168.7 | 168.5 |
| Chain distribution (%) | Actual measurement* | 77.7 | 14.5 | 0.4 | 2.1 | 3.1 | 0.8 | 0.3 | 1.2 |
| | Calculated value** | 77.6 | 13.4 | 0.6 | 3.1 | 3.1 | 1.1 | 0.1 | 1.1 |

* Determined from $^{13}C$-NMR spectrum

** Chain distribution expected in the case of a random copolymer

Table 4

| Example | Carbon source: Sort | Carbon source: g/ℓ*1 | Weight of dry cell g/ℓ*1 | Copolymer content in dry cells % | Copolymer composition mol%: [3HB] | [3HV] | [3HP] | [5HV] | Molecular weight*2: Mw $x10^3$ | Mw/Mn | Melting temperature Tm °C | Crystallinity Xc % |
|---|---|---|---|---|---|---|---|---|---|---|---|---|
| Example 4 | δ-valerolactone<br>Propionic acid | 80<br>8 | 53 | 25 | 91 | 4 | 4 | 1 | 360 | 1.2 | 126 | 60 |
| Example 5 | δ-valerolactone<br>Propionic acid | 44<br>36 | 57 | 29 | 88 | 8 | 3 | 1 | 290 | 1.2 | 124 | 55 |
| Example 6 | δ-valerolactone<br>Propionic acid | 71<br>71 | 69 | 39 | 77 | 19 | 3 | 1 | 760 | 1.4 | 124 | 54 |

*1 g/ℓ- Medium (culture broth)
*2 Mw: Weight average molecular weight
Mn: Number average molecular weight

According to the production process of the present invention, a novel four-unit random copolymer (polyester) comprising four kinds of monomer unit therein particularly from one kind of carbon source, using as a carbon source δ-valerolactone, 1,5-pentanediol and/or mono- or bis-carboxylic acid ester of 1,5-pentanediol, can be obtained.

The copolymer according to the present invention has a crystallinity of 54-64%, which is much lower

than that of a copolymer comprising PHB (70% or more), has a satisfactory flexibility, and has a melting temperature of 120 to 130 ° C. This value is, of course, 140 ° C or less, which is considered to be advantageous for the thermal molding (Polymer Preprints, Japan pamphlet 1989, 38, 2740-2742). Still further, it has a sufficient heat resistance when effecting a thermal disinfection (105-110 ° C).

The melting temperature of the copolymer according to the present invention is not readily affected by each monomer unit component. That is, even if the ratio of each component varies considerably, the melting temperature still remains in the preferable temperature range of 120 to 130 ° C.

In the bipolymer comprising 3HB and 3HV mentioned in the item of the prior art and the subject thereof (Japanese Unexamined Patent Publication (Kokai) No. 58-69224, Example 4), even if 3HV constructs 24 mol% of the copolymer, the melting temperature does not fall to about 180 ° C-170 ° C; 180 ° C is the melting temperature of PHB. In the present invention, when the amount of the 3HV component is 19 mol%, the melting temperature falls to 124 ° C (Example 6).

In the terpolymer comprising 3HB, 3HV and 5HV components (Japanese Unexamined Patent Publication (Kokai) No. 64-48820), it is shown that, in 63 mol% of the 3HV component and 11 mol% of the 5HV component, the melting point is 101 ° C, but in the present invention, at 2% of the 5HV component, the melting point falls to a preferable temperature range of 120 to 130 ° C. In the above-mentioned bipolymer comprising 3HB and 3HV (Japanese Unexamined Patent Publication (Kokai) No. 58-69224, it is stated that the amount of the 3HV component which lowers the melting temperature to 101 ° C is 32 mol% or 60-mol%, and thus it can be considered that the considerable drop in the melting temperature is greatly contributed to by the 3HV component content. The wide allowance range which can provide an appropriate melting temperature range of 120 to 130 ° C is a noticeable characteristic of the four-unit random copolymer according to the present invention.

In the conventional production processes of a copolymer using a microorganism, valeric acid, which is relatively expensive, is often used. In these processes, usually the yield of the microorganism cell is several grams - ten to twenty grams/ℓ. In the present invention, however, a much higher yield of the microorganism cell of 35-70g/ℓ is obtained, using as a carbon source δ-valerolactone, 1,5-pentanediol or the carboxylic acid ester of 1,5-pentanediol, which are not expensive. Therefore, the present process is industrially advantageous in that the polymer production rate is high.

The copolymer of the present invention having an excellent moldability is expected to be able to be applied for medical materials such as surgical thread, broken bone setting materials, slow release preparations, etc., hygienic materials such as diapers, sanitary articles, etc., agricultural and horticultural materials such as multi films, slow release chemicals, etc., fishery materials such as fishing nets, etc., packaging materials, and others.

## Claims

1. A random copolymer comprising, as repeating units,

(i) 50 to 97 mol% of 3-hydroxybutyrate unit (3HB) having the formula (I):

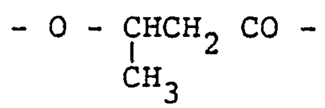

(I)

(ii) 1 to 25 mol% of 3-hydroxyvalerate unit (3HV) having the formula (II):

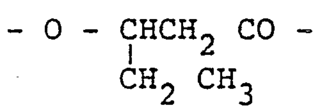

(II)

(iii) 1 to 15 mol% of 3-hydroxypropionate unit (3HP) having the formula (III):

$- OCH_2 CH_2 CO -$ (III)

and

(iv) 1 to 10 mol% of 5-hydroxyvalerate unit (5HV) having the formula (IV):

- $OCH_2$ $CH_2$ $cH_2$ $CH_2$ CO - (IV)

wherein the total of the units 3HB, 3HV, 3HP and 5HV is 100 mol%, and having a weight average molecular weight within the range of from 10,000 to 2,500,000.

2. A random copolymer as claimed in claim 1, wherein the copolymer comprises (i) 65 to 97 mol% of 3HB, (ii) 1 to 20 mol% of 3HV, (iii) 1 to 10 mol% of 3HP, and (iv) 1 to 5 mol% of 5HV.

3. A random copolymer as claimed in claim 1 or 2, wherein the weight average molecular weight of the random copolymer is 100,000 to 1,500,000.

4. A process for producing a random copolymer according to any of the claims 1 to 3, comprising culturing a microorganism capable of producing a polyhydroxyalkanoate under at least one restricted condition of the amounts of nitrogenous and phosphorous source in the presence of at least one component selected from the group consisting of δ-valerolactone, 1,5-pentanediol and mono- and bis-carboxylic acid esters of 1,5-pentanediol, to form and accumulate a random copolymer comprising 3-hydroxybutyrate units (3HB), 3-hydroxyvalerate units (3HV), 3-hydroxypropionate units (3HP) and 5-hydroxyvalerate units (5HV) within the microorganism cells, followed by recovering the same.

5. A process as claimed in claim 4, wherein said microorganism belongs to the genus Alcaligenes.

6. A process as claimed in claim 4, wherein said microorganism is at least one member selected from the group consisting of those belonging to Alcaligenes facecalis, Alcaligenes ruhlandii, Alcaligenes latus, Alcaligenes aquamarinus and Alcaligenes eutrophus.

7. A process as claimed in any of the claims 4 to 6, wherein said culturing is aerobically effected under the conditions of a temperature of 20 to 40 °C and a pH of 6 to 10.